# FASCICULE DE BREVET EUROPEEN

(11) **EP 2 038 244 B1**
(45) Date de publication et mention de la délivrance du brevet: **13.02.2013**
(21) Numéro de dépôt: 07803787.6
(22) Date de dépôt: 27.06.2007
(51) Int. Cl.: C07C 37/14, C07C 39/08

(54) **PROCÉDÉ DE SÉPARATION ET DE PURIFICATION D'HYDROQUINONE À PARTIR DE MÉLANGES BRUTS**
VERFAHREN ZUM TRENNEN UND REINIGEN VON HYDROCHINON AUS ROHGEMISCHEN
PROCESS FOR SEPARATING AND PURIFYING HYDROQUINONE FROM CRUDE MIXTURES

(30) Priorité: 29.06.2006 FR 0605870
(43) Date de publication de la demande: 25.03.2009
(73) Titulaire: Rhodia Opérations, 93306 Aubervilliers (FR)
(72) Inventeur: GAYET, Hubert, 69800 Saint Priest (FR); HEINISCH, Bruno, F-69100 Villeurbanne (FR)
(74) Mandataire: Dutruc-Rosset, Marie-Claude
(86) Numéro de dépôt international: PCT/FR2007/001078
(87) Numéro de publication internationale: WO 2008/000954

(56) Documents cités:
- FR-A1- 2 788 763

## Description

La présente invention a trait à un procédé de séparation et de purification, permettant d'obtenir de l'hydroquinone purifiée à partir de mélanges bruts contenant essentiellement de l'hydroquinone, en association avec de très faibles quantités d'impuretés, typiquement présentes à raison de 0,5 à 4 % en masse.

L'invention concerne également les dispositifs, et notamment les installations de taille industrielle, permettant la mise en oeuvre de ce procédé.

L'hydroquinone (ou 1,4-dihydroxybenzène), est un composé bien connu, issu de l'industrie du phénol, où il est généralement obtenu en mélange avec divers autres composés dihydroxylés ou trihydroxylés du benzène, tels que le pyrocatéchol (1,2-dihydroxybenzène, désigné aussi par pyrocatéchine), le résorcinol (1,3-dihydroxybenzène, désigné aussi par résorcine), ou encore le pyrogallol (1,2,3-trihydroxybenzène).

Une des voies de synthèse de l'hydroquinone consiste à effectuer l'hydroxylation du phénol par le peroxyde d'hydrogène, notamment en présence de catalyseurs acides homogènes ou hétérogènes.

Ainsi, on peut faire appel comme selon FR 2 071 464, a un acide protique fort c'est-à-dire un acide présentant un pKa dans l'eau inférieur à 0,1, de préférence inférieur à -1.

Comme exemples d'acides protiques forts, on peut mentionner entre autres, l'acide sulfurique, l'acide chlorosulfurique, l'acide perchlorique, les acides sulfoniques comme par exemple, l'acide méthanesulfonique, trifluorométhanesulfonique, toluènesulfonique, phénolsulfonique.

Comme autres exemples de catalyseurs acides protiques, on peut citer les résines sulfoniques et plus particulièrement les résines commercialisées sous différentes dénominations commerciales. On peut citer, entre autres, les résines suivantes: TEMEX 50, AMBERLYST 15, AMBERLYST 35, AMBERLYST 36, DOWEX 50W.

Les résines précitées sont constituées d'un squelette polystyrénique qui porte des groupes fonctionnels qui sont des groupes sulfoniques. Le squelette polystyrénique est obtenu par polymérisation du styrène et du divinylbenzène, sous l'influence d'un catalyseur d'activation, le plus souvent un peroxyde organique, ce qui conduit à un polystyrène réticulé qui est ensuite traité par de l'acide sulfurique ou sulfochlorique concentré conduisant à un copolymère styrène-divinylbenzène sulfoné.

Il est également possible de faire appel à des résines sulfoniques qui sont des copolymères phénol-formol et qui portent sur le noyau aromatique un groupe méthylènesulfonique, par exemple la résine commercialisée sous la dénomination DUOLITE ARC 9359.

D'autres résines disponibles sur le marché conviennent également et l'on peut citer les résines perfluorées porteuses de groupes sulfoniques et, plus particulièrement le NAFION qui est un copolymère de tétrafluoroéthylène et de perfluoro[2-(fluorosulfonyléthoxy)-propyl] vinyl éther.

Comme autres catalyseurs convenant dans les procédés d'hydroxylation, on peut mentionner les complexes de fer Il et de cuivre Il (FR 2 121 000, USSR 1 502 559) et tout autre catalyseur de type Fenton.

D'autres procédés de préparation de l'hydroquinone font appel à une catalyse hétérogène. Ainsi, on peut mettre en oeuvre une zéolithe acide de type silicalite de titane (ou titanosilicalite-1) ou de fer de type TS-1 (FR 2 489 816), une zéolithe de type silicalite de titane MEL (EP 1 131 264) ou une titanozéosilite de type MFI (EP 1 123 159). Il est également possible d'utiliser une zéolithe MCM-22 (FR 2 856 681).

A l'issue de telles réactions d'hydroxylation, on obtient un mélange comprenant essentiellement du pyrocatéchol et de l'hydroquinone, en proportions variables avec en général, un ratio massique pyrocatéchol/hydroquinone de l'ordre de 0,25 à 4,0, ainsi que différents sous-produits en des quantités beaucoup plus faibles, notamment du résorcinol et du pyrogallol, généralement à des teneurs de l'ordre de 0,5 à 4,0 % en masse, pourcentages exprimés par rapport à la quantité d'hydroquinone et de pyrocatéchol formée.

On obtient des mélanges de compositions variables comprenant, en masse, de 20 à 80% de pyrocatéchol, de 80 à 20 % d'hydroquinone, de 0,1 à 2 % de résorcinol et de 0,1 à 2 % de pyrogallol.

Typiquement, on obtient des mélanges comprenant, en masse, de 50 à 80 % de pyrocatéchol, de 20 à 50 % d'hydroquinone, de 0,1 à 2 % de résorcinol et de 0,1 à 2 % de pyrogallol.

Pour isoler l'hydroquinone à partir de mélanges bruts de ce type, une méthode actuellement connue consiste à effectuer la distillation dudit mélange permettant d'obtenir en tête de distillation le pyrocatéchol (qui est le composé le plus volatil du mélange), et en pied de distillation, une "hydroquinone brute", à savoir un mélange comprenant essentiellement de l'hydroquinone, associée à de faibles quantités d'impuretés (notamment résorcinol et pyrogallol ainsi que d'éventuelles traces dé pyrocatéchol non éliminées par la distillation).

La demande de brevet FR 2.788.763 divulgue un procédé de séparation et de purification de mélanges bruts contenant essentiellement de l'hydroquinone, du résorcinol et des goudrons afin d'en extraire d'une part l'hydroquinone et d'autre part le résorcinol. Ce procédé comprend au moins deux étapes de distillation en tête desquelles (tête des colonnes) un mélange hydroquinone/résorcinol est obtenu avant d'être soumis à une étape de raffinage réalisée en quatre phases pour la purification de chacun des mélanges hydroquinone/résorcinol.

Le problème à la base de l'invention est donc de fournir un procédé économique permettant la purification d'une hydroquinone brute du type précité, à savoir contenant essentiellement de l'hydroquinone en association avec de très faibles quantités d'impuretés de natures différentes incluant du résorcinol et du pyrogallol.

La purification de l'hydroquinone pose plusieurs problèmes à l'Homme du Métier.

L'hydroquinone est un composé qui présente une température d'ébullition très élevée supérieure à 200°C même sous pression réduite de 100 hPa.

De plus, l'hydroquinone est sensible à la dégradation thermique et conduit à des produits de dégradation colorés.

Dans l'hydroquinone brute à purifier selon l'invention, il y a essentiellement de l'hydroquinone c'est-à-dire au moins 90 % en masse d'hydroquinone et le reste étant constitué par les impuretés à éliminer. Préférentiellement, l'hydroquinone brute comprend au moins 96 % en masse d'hydroquinone.

Or, il y a une difficulté grande à éliminer des teneurs extrêmement faibles d'impuretés.

La difficulté est par ailleurs accrue de par la nature des impuretés. Les composés à séparer ont des volatilités proches car il y a présence d'isomères de l'hydroquinone et il y a également présence de pyrogallol parmi les impuretés à éliminer.

La séparation du pyrogallol présent dans le mélange des impuretés à éliminer posait problème à l'Homme du Métier. En effet, le pyrogallol est un composé qui se dégrade thermiquement encore plus facilement que l'hydroquinone et sa décomposition conduit à des impuretés colorées.

Le problème qui se posait était de savoir si l'hydroquinone pouvait être débarrassée des impuretés colorées générées par la décomposition thermique de tout ou partie du pyrogallol.

Il a maintenant été trouvé et c'est ce qui constitue l'objet de la présente invention, un procédé de purification d'une hydroquinone brute *HQ⁰* contenant essentiellement de l'hydroquinone et de faibles quantités d'impuretés incluant à la fois :
(i) des impuretés ayant une température de vaporisation inférieure à celle de l'hydroquinone, dites ci-après "*impuretés légères*", incluant en particulier du résorcinol, de préférence à titre d'impureté majoritaire parmi les impuretés légères ; et
(ii), des impuretés ayant une température de vaporisation supérieure à celle de l'hydroquinone, désignées ci-après par "*impuretés lourdes*", incluant en particulier du pyrogallol, de préférence à titre d'impureté majoritaire parmi les impuretés lourdes,
ledit procédé étant caractérisé par le fait qu'il comprend les étapes suivantes :
(A) une distillation d'étêtage, dans laquelle on injecte l'hydroquinone brute *HQ*⁰ dans une colonne de distillation opérant à un taux de reflux compris entre 300 et 2000 et où on élimine le résorcinol en tête de distillation éventuellement conjointement à tout ou partie des autres impuretés légères, ce par quoi on récupère en pied de colonne un mélange brut M comprenant de l'hydroquinone et les impuretés lourdes ; et
(B) une distillation d'équeutage dans laquelle on injecte le mélange brut M obtenu dans l'étape (A) dans une colonne de distillation et où on élimine le pyrogallol en pied de colonne éventuellement conjointement à tout ou partie des autres impuretés lourdes, ce par quoi on récupère en tête de colonne de l'hydroquinone sous une forme purifiée (*HQ*) comprenant moins de 4000 ppm d'impuretés.

Les inventeurs ont maintenant trouvé que la mise en oeuvre des étapes successives de distillation (A) et (B) ci-dessus permet d'obtenir une purification efficace d'une hydroquinone brute comprenant un mélange de composés plus volatils que l'hydroquinone et de composés moins volatils que l'hydroquinone, de type résorcinol et pyrogallol avec obtention d'une hydroquinone purifiée avec des quantités d'impuretés résiduaires extrêmement faibles.

Dans ce cadre, il est à noter que le procédé de l'invention peut être utilisé pour traiter en particulier des hydroquinones brutes contenant essentiellement de l'hydroquinone, à raison d'au moins 90 %, typiquement à raison de 96 à 99,5 % en masse et de l'ordre de 0,5 à 4 % en masse d'impuretés, par exemple des quantités d'impuretés aussi faibles que de 0,5 à 2 % en masse, pour conduire *in fine* à des hydroquinones purifiées contenant en général moins de 4000 ppm d'impuretés, le plus souvent moins de 3000 ppm. De façon plus spécifique, le procédé de l'invention peut notamment être mis en oeuvre pour la préparation d'hydroquinones de haute pureté, contenant des impuretés à une teneur de moins de 2500 ppm, typiquement au plus de l'ordre de 2000 ppm, de préférence au plus de l'ordre de 1500 ppm, et plus préférentiellement au plus de l'ordre de 1000 ppm, voire moins.

La possibilité d'une telle efficacité de séparation s'avère relativement inattendue, dans la mesure où le problème qui se posait en termes de séparation était particulièrement difficile à résoudre, notamment compte tenu du fait que les différents composés à séparer ont des volatilités relatives très proches. En outre l'hydroquinone présente une haute température de fusion (172,5°C) ainsi qu'une température de vaporisation très élevée, même sous pression réduite (258°C sous 500 hPA; 208°C sous 100 hPa).

De façon encore plus inattendue, il s'avère que, bien que l'hydroquinone soit sensible à la dégradation thermique et qu'il soit nécessaire de maintenir l'hydroquinone à des températures de 170 à 220°C tout au long de la distillation des étapes (A) et (B), ces étapes peuvent néanmoins être conduites de façon efficace tout en limitant les phénomènes de dégradation thermique de l'hydroquinone, qui sont susceptibles de former des produits de dégradation colorés de type quinones.

Dans ce cadre, les inventeurs ont en particulier maintenant mis en évidence que les étapes (A) et (B) peuvent être conduites efficacement en limitant néanmoins le temps de séjour dans les colonnes de distillation, ce par quoi les phénomènes de dégradation thermique peuvent être très sensiblement inhibés.

Ces phénomènes de dégradation peuvent en outre encore être davantage évités en limitant la présence d'oxygène dans les colonnes de distillation, par exemple en travaillant sous atmosphère inerte.

En outre, les travaux des inventeurs ont permis d'établir que, dans les conditions du procédé de l'invention, dans le cas où d'éventuels produits de dégradation thermiques colorés de l'hydroquinone de type quinones se forment, ceux-ci sont substantiellement récupérés en pied de colonne de la distillation d'équeutage de l'étape (B).

Selon la présente invention, il a également été découvert que le pyrogallol et les impuretés résultant de sa dégradation pouvaient être éliminées d'une manière efficace au cours d'une opération de distillation.

Comme mentionné précédemment, le pyrogallol se décompose au moins partiellement en impuretés colorées du fait que la distillation de l'hydroquinone est effectuée à température élévée.

Il était à craindre que les impuretés colorées se retrouvent, dans l'étape (B) en tête de distillation avec l'hydroquinone.

Il a été trouvé selon l'invention que le pyrogallol ainsi que les impuretés formées par sa dégradation restaient en pied de distillation et pouvaient donc - être efficacement séparées de l'hydroquinone.

- L'invention fournit un procédé de purification permettant une élimination à la fois efficace et économique des différentes impuretés.

A l'issue des étapes (A) et (B), l'hydroquinone purifiée est obtenue directement sous une forme liquide.

La composition exacte de l'hydroquinone brute *HQ⁰* traitée selon les étapes (A) et (B) du procédé de l'invention peut varier en une assez large mesure, le procédé de l'invention se révélant toutefois surtout intéressant pour des hydroquinones brutes contenant essentiellement de l'hydroquinone à raison d'au moins 90 % en masse et de faibles quantités d'impuretés inférieures à 10 % en masse. Les impuretés majoritaires sont le résorcinol et le pyrogallol : le pyrocatéchol représentant moins de 1 % de la masse d'impuretés. Le ratio pondéral pyrogallol/résorcinol varie généralement entre 0,2 et 5.

Le procédé de l'invention est particulièrement intéressant pour traiter des hydroquinones brutes comprenant de l'hydroquinone à raison de 96 à 99,5 % en masse, et des teneurs en impuretés de l'ordre de 0,5 à 4%, par exemple de 0,5 à 2 % en masse, notamment de 1 à 2 % en masse par rapport à la masse totale de l'hydroquinone brute.

Typiquement, une hydroquinone brute *HQ⁰* traitée selon l'invention contient de 0,1 à 2 %, par exemple de 0,2 à 1 % en masse, d'impuretés légères (ayant une température de vaporisation inférieure à celle de l'hydroquinone), incluant du résorcinol. Le résorcinol est en général une impuretés majoritaire au sein des impuretés légères, les impuretés légères comprenant en règle générale au moins 50 % en masse de résorcinol par rapport à la masse totale des impuretés légères, par exemple au moins 70 %, notamment au moins 80 %, en particulier au moins 90 % en masse, voire plus. Outre le résorcinol, les impuretés légères présentes dans l'hydroquinone brute *HQ⁰* peuvent notamment comprendre du pyrocatéchol.

Par ailleurs, dans l'hydroquinone brute *HQ⁰*, la quantité d'impuretés lourdes (ayant une température de vaporisation supérieure à celle de l'hydroquinone) est usuellement de 0,1 à 2 %, par exemple de 0,2 à 1 % en masse. Ces impuretés lourdes incluent en particulier du pyrogallol, généralement à titre d'impureté lourde majoritaire, en général en association avec d'autres impuretés lourdes, notamment des goudrons ou bien encore des produits de dégradation thermique de l'hydroquinone tels que des quinones. Ainsi, les impuretés lourdes comprennent en général au moins 50 % en masse de pyrogallol par rapport à la masse totale des impuretés lourdes, par exemple au moins 70 %, voire au moins 80 %, en particulier au moins 90 % en masse ou plus.

Selon un mode de réalisation particulier, l'hydroquinone brute *HQ⁰* traitée selon les étapes (A) et (B) est obtenue, ou susceptible d'être obtenue, à partir d'un mélange réactionnel issu d'une hydroxylation de phénol par le peroxyde d'hydrogène en présence de catalyseurs acides du type cité plus haut dans la présente description, après élimination substantielle du pyrocatéchol par distillation.

Une hydroquinone brute *HQ⁰* adaptée au procédé de l'invention comprend, en masse par rapport à la quantité totale d'hydroquinone brute :
- de 96 à 99,5 % d'hydroquinone,
- de 0,1 à 2 %, de préférence de 0,2 à 1 % de résorcinol,
- de 0,1 à 2 %, de préférence de 0,2 à 1 % de pyrogallol
- éventuellement du pyrocatéchol sous forme de traces, typiquement à une teneur inférieure à 500 ppm (0,05 %), de préférence inférieure à 100 ppm (0,01 %).

Quelle que soit la nature exacte de l'hydroquinone brute *HQ⁰* traitée selon le procédé de l'invention, les étapes de distillation (A) et (B) sont avantageusement mises en oeuvre dans les conditions exposées ci-après.

L'étape (A) de distillation d'étêtage vise à éliminer le résorcinol et de préférence sensiblement toutes les impuretés légères présentes dans l'hydroquinone brute *HQ⁰*, en les entraînant en tête de colonne, pour récupérer en pied de colonne un mélange brut M comprenant essentiellement de l'hydroquinone et des impuretés lourdes, appauvri en impuretés légères, en particulier en résorcinol.

Il est à noter que l'élimination des impuretés en tête de colonne s'accompagne en général d'un départ d'une fraction d'hydroquinone en tête de colonne, qui n'est donc pas récupérée dans le mélange brut M qui sera mis en oeuvre dans l'étape (B). Pour limiter cette perte d'hydroquinone dans l'étape (A), on peut notamment jouer sur le nombre d'étages théoriques et le taux de reflux de la colonne de distillation utilisée dans l'étape (A), ce par quoi on peut typiquement obtenir dans le procédé un ratio (hydroquinone perdue/hydroquinone dans le mélange M) inférieur à 2 %, par exemple compris entre 0,2 et 1 %, notamment entre 0,3 et 0,7 %.

Le débit d'alimentation de l'hydroquinone brute *HQ⁰* dans la colonne de distillation de l'étape (A) peut varier en une assez large mesure, notamment en fonction du dimensionnement choisi pour la colonne et du débit recherché *in fine* pour l'hydroquinone purifiée. A titre non limitatif, on peut simplement préciser qu'il est possible de travailler avec des débits d'alimentation allant jusqu'à 3000 kg/h, voire jusqu'à 5000 kg/h. Typiquement, on peut employer des débits de l'ordre de 100 à 3000 kg/h.

Dans l'étape (A), le point d'alimentation où est introduit l'hydroquinone brute *HQ⁰* est en général sensiblement à mi-hauteur de la colonne de distillation, à savoir avec un rapport volumique de la zone de rectification de la colonne de l'étape (A) à la zone d'épuisement de la colonne de l'étape (A) en général compris entre 25 : 75 et 75 : 25, plus préférentiellement entre 30 : 70 et 70 : 30, par exemple entre 40 : 60 et 60 : 40. On entend ici par "zone de rectification" le volume interne de la colonne de distillation de l'étape (A) qui est situé au-dessus du plan horizontal contenant le point d'alimentation, par opposition à la "zone d'épuisement", correspondant au volume interne de colonne situé en dessous de ce plan horizontal.

Le flux qui sort en tête de la colonne de distillation de l'étape (A), qui contient essentiellement les impuretés légères à éliminer et un peu d'hydroquinone, peut avantageusement être dérivé en partie, pour être réinjecté dans la colonne de distillation, selon la technique du reflux. La quantité de flux qui est réinjecté dans la colonne peut être quantifiée par un taux de reflux, défini par le rapport du débit sortant effectivement à la sortie de la tête de colonne, rapporté au débit de matière réinjectée de la tête de la colonne vers l'intérieur de la colonne. Dans la colonne de la distillation d'étêtage de l'étape (A), ce taux de reflux est compris entre 300 et 2000, typiquement entre 400 et 1500, par exemple entre 500 et 1000.

Par ailleurs, le nombre d'étages théoriques de la colonne utilisée dans l'étape (A) est avantageusement d'au moins 20, de préférence d'au moins 30, par exemple entre 30 et 50.

D'autre part, le temps de séjour de l'hydroquinone dans la colonne de l'étape (A) est de préférence inférieur à 1 heure, de préférence inférieur à 45 minutes, et plus préférentiellement encore inférieur à 30 minutes, ce qui permet notamment d'inhiber les phénomènes de.dégradation thermique de l'hydroquinone, en limitant le temps pendant lequel celle-ci est soumise à haute température. Néanmoins, ce temps de séjour reste en général au moins égal à 10 minutes, par exemple d'au moins 15 minutes notamment pour permettre une séparation efficace des impuretés légères dans l'étape (A). On obtient ainsi un bon compromis dans l'étape (A) entre séparation et inhibition de la dégradation thermique avec des temps de séjour typiquement de l'ordre de 15 à 30 minutes.

L'étape (B) qui suit l'étape (A) consiste quant à elle en une distillation d'équeutage, qui vise à éliminer le pyrogallol présent dans le mélange brut M obtenu à l'issue de la distillation d'étêtage, et de préférence sensiblement toutes les impuretés lourdes. Dans l'étape (B), à l'inverse de l'étape (A), ce sont les impuretés qui sont envoyées en pied de colonne, et c'est en tête de colonne que l'on récupère l'hydroquinone sous forme purifiée.

Là également, l'élimination des impuretés vers le pied de colonne s'accompagne d'un départ d'une partie de l'hydroquinone en pied de colonne. Ainsi, on ne récupère pas la totalité de l'hydroquinone du mélange M en tête de colonne. Pour limiter cette perte d'hydroquinone en pied de colonne de distillation de l'étape (B), on peut notamment jouer sur le taux de reflux et sur le nombre d'étages théoriques de la colonne de distillation de l'étape (B), ce par quoi on peut typiquement obtenir dans l'étape (B) un ratio (hydroquinone perdue/hydroquinone récupérée dans l'hydroquinone purifiée) inférieur à 2 %, par exemple compris entre 0,2 et 1 %.

Dans l'étape (B), le point d'alimentation où est introduit le mélange brut M issu du pied de colonne de l'étape (A) est en général sensiblement à mi-hauteur de la colonne de distillation. Typiquement, pour la colonne de l'étape (B), le rapport volumique de la zone de rectification à la zone d'épuisement est compris entre 25:75 et 75:25, plus préférentiellement entre 30:70 et 70 : 30, par exemple entre 40 : 60 et 60 : 40. Là encore, on entend par "zone de rectification" le volume interne de la colonne de distillation de l'étape (B) qui est situé au-dessus du plan horizontal contenant le point d'alimentation, par opposition à la "zone d'épuisement", correspondant au volume interne de la colonne situé en dessous de ce plan horizontal.

Par ailleurs, comme dans l'étape (A), on travaille avantageusement à reflux dans l'étape (B), à savoir en dérivant une partie du flux qui sort en tête de la colonne de distillation de l'étape (B), qui contient de l'hydroquinone purifiée, pour le réinjecter dans la colonne de distillation. Le taux de reflux dans la colonne de la distillation d'équeutage de l'étape (B), défini par le rapport du débit sortant effectivement à la sortie de la tête de colonne, rapporté au débit de matière réinjectée de la tête de la colonne vers la colonne, est avantageusement compris entre 1 et 15, typiquement entre 3 et 12, par exemple entre 4 et 10.

Par ailleurs, le nombre d'étages théoriques de la colonne utilisée dans l'étape (B) est avantageusement au moins égal à 20, de préférence au moins égal à 30, par exemple entre 30 et 50.

Dans l'étape (B), il est tout particulièrement important de contrôler le temps de séjour de l'hydroquinone dans la colonne de distillation.

D'une part, pour éviter la dégradation thermique du produit, il est en général avantageux de choisir un temps de séjour inférieur à 1 heure, plus avantageusement inférieur à 30 minutes dans la colonne de l'étape (B). Pour obtenir une séparation efficace des impuretés lourdes dans l'étape (B), il est toutefois préférable, le plus souvent, de travailler avec des temps de séjour de l'hydroquinone dans la colonne de l'étape (B) d'au moins 10 minutes, par exemple entre 15 et 30 minutes.

De façon plus générale, il est à noter que les étapes (A) et (B) sont conduites dans des conditions permettant la distillation de l'hydroquinone, ce qui implique notamment qu'elles sont conduites à des températures suffisantes pour que l'hydroquinone se présente à l'état liquide ou gazeux. Il est indiqué d'éviter la présence de tout point froid en deçà de 170°C (température de solidification de l'hydroquinone) dans le dispositif de mise en oeuvre des étapes de distillations (A) et (B), ce qui pourrait induire des phénomènes d'encrassement des colonnes, nuisibles au rendement et/ou à la qualité de la distillation, voire des phénomènes de prise en masse, qui nécessiteraient un arrêt complet du procédé et des opérations onéreuses de nettoyage de l'installation. A cet effet, par sécurité, on préfère en général que toutes les zones internes des colonnes de distillation mises en oeuvre pour les étapes (A) et (B) soient au minimum à une température de 175°C, et de préférence à une température d'au moins 180°C, par exemple d'au moins 185°C. La plupart des zones sont supérieures à ces températures, pour parvenir à la vaporisation de l'hydroquinone nécessaire à la distillation, la température restant néanmoins typiquement inférieure à 220°C.

En règle générale, pour obtenir les hautes températures requises, sans présence de points froids, on met avantageusement en oeuvre des colonnes à double enveloppe, en faisant circuler un fluide caloporteur porté à une température de l'ordre de 180 à 220°C. Comme fluides caloporteurs appropriés, on peut notamment citer les esters lourds d'acides carboxyliques, comme le phtalate d'octyle, les éthers aromatiques tels que l'oxyde de biphényle et/ou l'oxyde de benzyle, le diphényle, les terphényles, les autres polyphényles éventuellement partiellement hydrogénés, les huiles paraffiniques et/ou naphténiques, ou bien encore certains résidus de distillation du pétrole.

Il est par ailleurs souhaitable d'éviter au maximum d'établir des ponts thermiques entre le dispositif mis en oeuvre selon l'invention et le milieu extérieur, pour se prémunir de tout risque de déperditions thermiques.

Par ailleurs, compte tenu des hautes températures utilisées, il est le plus souvent souhaitable d'éviter la présence d'oxygène dans les étapes de distillation des étapes (A) et (B), notamment pour éviter une dégradation de l'hydroquinone en quinones. A cet effet, ces étapes sont avantageusement conduites sous une atmosphère inerte sensiblement exempte d'oxygène, par exemple sous azote ou bien encore sous argon, l'azote étant préféré notamment compte tenu de son coût réduit.

D'autre part, notamment pour éviter d'avoir à chauffer à des températures trop importantes, les distillations de chacune des étapes (A) et (B) sont avantageusement conduites sous pression réduite, ces pressions, identiques ou différentes dans les colonnes de distillation des étapes (A) et (B) sont typiquement comprises entre 50 et 100 hPA, par exemple entre 60 et 90 hPa. Les pressions de travail des étapes (A) et (B) peuvent être identiques ou différentes.

La distillation des étapes (A) et (B) peut avantageusement être conduite selon un mode continu, notamment en injectant à débit constant l'hydroquinone brute *HQ⁰* à traiter à l'entrée de la colonne de distillation d'étêtage. Il n'est toutefois pas exclu de la conduire selon un mode discontinu.

Quel que soit leur mode exact de mise en oeuvre, les étapes (A) et (B) conduisent *in fine,* en tête de la colonne de l'étape (B), à une hydroquinone purifiée *HQ*, obtenue après condensation à l'état liquide, qui ne pose pas les problèmes de manipulation rencontrés avec les poudres d'hydroquinone.

L'hydroquinone purifiée *HQ* obtenue à l'état liquide à l'issue des étapes (A) et (B) est en général refroidie, de préférence sous une atmosphère de gaz inerte sensiblement exempte d'oxygène (azote ou argon, par exemple) pour éviter une dégradation de l'hydroquinone, et mise en forme à l'état d'objets solides de taille adaptée à une manipulation sans risques de poussiérage, typiquement de l'ordre d'au moins quelques centaines de microns à quelques millimètres.

Cette mise en forme peut notamment être effectuée en mettant en oeuvre l'une ou l'autre des techniques suivantes :
- un écaillage sur cylindre ou sur bande, dans lequel l'hydroquinone liquide est mise en contact avec un cylindre ou une bande métallique plus froide, puis en raclant avec un couteau le film obtenu sur le cylindre, ce par quoi on récupère l'hydroquinone solide sous forme d'écailles,
- un "prilling", tel que décrit notamment dans EP-A 1556322, dans lequel on disperse l'hydroquinone liquide sous forme de gouttes dans un courant d'air, par exemple en la faisant tomber du haut d'une tour dans une colonne d'air froid, ce qui conduit à une obtention de l'hydroquinone solide sous forme de billes,
- une trempe, où l'hydroquinone liquide est dispersée généralement sous forme de gouttes, dans un liquide froid non miscible, ce par quoi on obtient de l'hydroquinone solide sous forme de granulats.

Quelle que soit sa forme finale, l'hydroquinone purifiée obtenue selon le procédé de l'invention comprend un taux très réduit d'impuretés, moins de 4000 ppm d'impuretés, le plus souvent moins de 3000 ppm.

Selon un mode de réalisation particulier, le procédé de l'invention peut être mis en oeuvre pour la préparation d'hydroquinone de haute pureté, contenant typiquement moins de 2500 ppm, préférentiellement moins de 2000 ppm d'impuretés. Une telle hydroquinone de haute pureté contient avantageusement moins de 2000 ppm d'impuretés légères telles que du résorcinol ou du pyrocatéchol (traces), cette teneur en impuretés légères étant de préférence inférieure à 1500 ppm, par exemple entre 1000 et 1500 ppm et plus préférentiellement entre 300 et 1000 ppm. La teneur en impuretés lourdes est quant à elle avantageusement inférieure à 500 ppm, de préférence inférieure à 300 ppm, par exemple entre 20 et 200 ppm.

Le procédé de l'invention permet d'éliminer efficacement les impuretés puisque les teneurs de résorcinol et de pyrogallol peuvent descendre respectivement jusqu'à 300 ppm et 20 ppm et le pyrocatéchol n'est plus détectable par l'analyse.

Les hydroquinones solides telles qu'obtenues selon l'invention présentent par ailleurs, une très faible teneur en produits de dégradation thermique, tels que des quinones, en particulier lorsque le procédé est conduit en évitant tout contact de l'hydroquinone chauffée avec l'oxygène. Cette faible teneur en produit de dégradation se traduit par une absence sensible de coloration de l'hydroquinone purifiée obtenue, qui a un aspect blanc. La coloration de l'hydroquinone peut être mesurée plus précisément, par analyse colorimétrique d'une solution aqueuse de ladite hydroquinone, typiquement en réalisant une solution à 5 % en masse à température ambiante. On peut obtenir pour les hydroquinones purifiées de l'invention des indices de colorimétrie faibles, compris par exemple entre 20 et 200 Hazen et plus préférentiellement entre 20 et 100 Hazen.

La présente invention décrit également un dispositif pour la mise en oeuvre du procédé de l'invention.

Ce dispositif, qui se présente le plus souvent sous la forme d'une installation de dimensions industrielles, comprend :
- une première colonne de distillation adaptée à la distillation d'étêtage d'une hydroquinone brute *HQ⁰* selon l'étape (A) précitée, conçue pour éliminer le résorcinol en tête de colonne et récupérer en pied de colonne un mélange contenant la majeure partie de l'hydroquinone et les impuretés lourdes le taux de reflux de ladite colonne étant compris entre 300 et 2000; et
- une deuxième colonne de distillation adaptée à la distillation d'équeutage de l'étape (B) précitée, dont l'entrée est reliée au pied de colonne de la première colonne, conçue pour éliminer en pied de colonne le pyrogallol contenu dans le mélange brut provenant du pied de colonne de la première colonne de distillation, et pour obtenir en tête de colonne l'hydroquinone sous forme purifiée.

Dans ce dispositif, les deux colonnes de distillation présentent avantageusement les caractéristiques préférentielles telles que définies plus haut dans la présente description. En particulier, les colonnes sont de préférence des colonnes à double enveloppe chauffées par un fluide caloporteur du type précité.

L'invention sera encore explicitée davantage par le biais de la description qui suit, faite en référence aux figures ci-annexées, où :

La Figure 1 est une représentation schématique du dispositif général utilisé selon l'invention pour la mise en oeuvre des étapes de distillation (A) et (B).

La Figure 2 est une représentation schématique d'un dispositif mettant en oeuvre une variante spécifique de l'invention, selon laquelle on prépare l'hydroquinone purifiée à partir d'un mélange tel qu'issu d'une réaction d'hydroxylation de phénol par du peroxyde d'hydrogène, en présence d'un catalyseur acide de type acide protique fort.

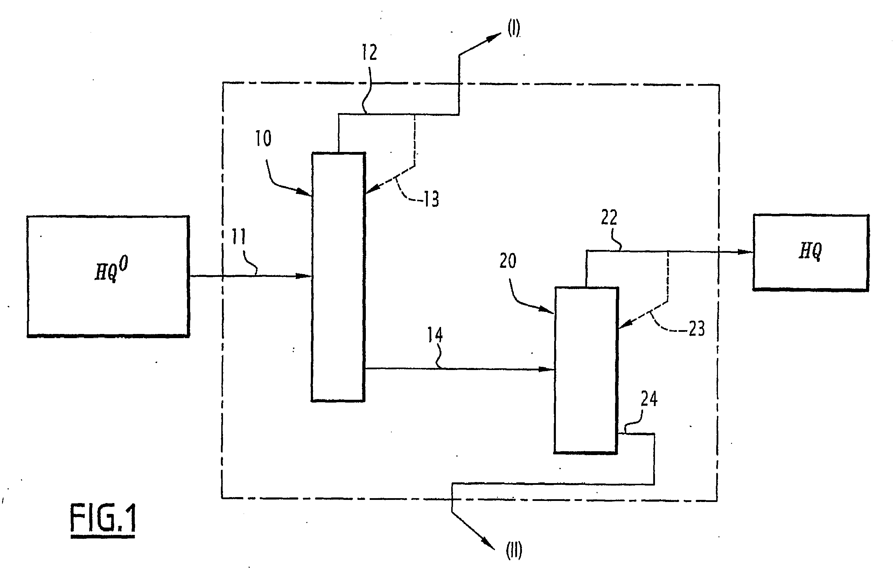

Sur la Figure 1, qui illustre le principe général du procédé de l'invention, on introduit l'hydroquinone brute *HQ⁰* (11) qui est un mélange d'hydroquinone, d'impuretés légères contenant du résorcinol et d'impuretés lourdes contenant du pyrogallol, dans une première colonne de distillation (10) : l'alimentation de la colonne (10) est de préférence localisée sensiblement à mi-hauteur de la colonne.

La distillation d'étêtage [étape (A)] qui s'opère dans la colonne (10) conduit, au niveau de la tête de colonne (10), à l'élimination d'un flux symbolisé par (I) sur la figure 1, comprenant du résorcinol et éventuellement d'autres impuretés légères, ainsi qu'une fraction de l'hydroquinone. Ce flux (I) venant de la tête de colonne peut être ensuite traité, recyclé ou éliminé, notamment par un traitement dans un brûleur. Le flux (12) qui sort en tête de colonne (10) est dérivé en partie (13) pour être réinjecté dans la colonne (10), généralement latéralement en tête de colonne, pour assurer le reflux dans la colonne, reflux qui est mis en place notamment pour améliorer l'efficacité de la séparation.

Parallèlement, on obtient au niveau du pied de colonne (10), un mélange brut M (14) contenant de l'hydroquinone et des impuretés lourdes, avec éventuellement des traces d'impuretés légères non extraites lors de l'étape précédente. Ce mélange brut M est introduit dans une deuxième colonne de distillation (20), l'alimentation de la colonne (20) étant de préférence sensiblement à mi-hauteur. Dans cette colonne (20), le mélange brut M issu de la colonne (10) va subir la distillation d'équeutage de l'étape (B).

Dans la colonne (20), la distillation conduit à la séparation d'une part, d'une hydroquinone purifiée *HQ* (22) récupérée en tête de colonne (20) et obtenue après condensation, sous forme liquide et d'autre part, d'un flux (24) symbolisé par (II) sur la figure 1, comprenant du pyrogallol et éventuellement d'autres impuretés lourdes (ainsi qu'un peu d'hydroquinone) qui sont évacués au niveau du pied de colonne (20). Ce flux (II) qui sort en pied de colonne (20) est ensuite traité, éliminé ou recyclé. Là encore, il est avantageux de mettre en place un reflux (23) en dérivant une partie du flux (22), notamment pour améliorer l'efficacité de la séparation des impuretés lourdes

On obtient en tête de la colonne (20), une hydroquinone (22) purifiée ne contenant essentiellement plus d'impuretés et qui a été liquéfiée après passage dans un condenseur.

Cette hydroquinone liquide est ensuite refroidie et mise en forme, par exemple par écaillage sur cylindre ou sur bande (cette étape n'étant pas représentée sur la figure).

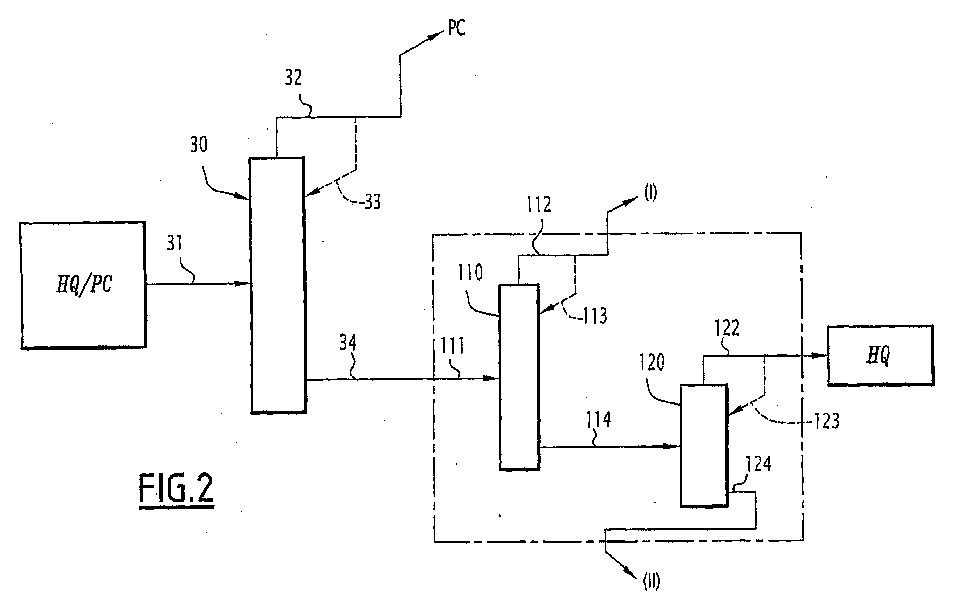

Sur la Figure 2, un dispositif du type précité est mis en oeuvre dans un procédé plus complexe de traitement d'un mélange *HQ*/*PC* du type obtenu à l'issu d'une hydroxylation de phénol par le peroxyde d'hydrogène en présence d'un catalyseur, ce mélange *HQ*/*PC* comprenant essentiellement du pyrocatéchol *(PC)* et de l'hydroquinone, et de faible quantités d'impuretés légères (résorcinol) et d'impuretés lourdes (pyrogallol).

Ce mélange *HQ*/*PC* (31) alimente une première colonne de distillation (30) destinée à éliminer substantiellement le pyrocatéchol (32) en tête de colonne. Là encore, il est avantageux de mettre en place un reflux (33) en dérivant une partie du flux (32). On obtient en tête de colonne 30, un pyrocatéchol purifié (32).

Quel que soit le mode de mise en oeuvre de la distillation dans la colonne (30), on obtient en pied de colonne (30), une hydroquinone brute *HQ⁰* (34) contenant essentiellement de l'hydroquinone (typiquement entre 96 et 99,5 %), associée à de faibles quantités d'impuretés, à savoir de l'ordre de 0,1 à 2 % d'impuretés légères (résorcinol et traces de pyrocatéchol) et de l'ordre de 0,1 à 2 % d'impuretés lourdes (pyrogallol pour l'essentiel),

Cette hydroquinone brute *HQ⁰* est ensuite soumise à un traitement de distillation d'étêtage dans la colonne (110), où les impuretés légères (résorcinol et traces de pyrocatéchol) sont éliminées en tête de colonne (110), sous la forme d'un flux symbolisé par (I) sur la figure 2. Le flux qui sort en tête de colonne (112) est dérivé en partie (113) pour être réinjecté dans la colonne (110) pour assurer le reflux. Au niveau du pied de colonne (110), on récupère un mélange brut (114) contenant de l'hydroquinone et les impuretés lourdes (pyrogallol essentiellement).

Le mélange brut ainsi obtenu est introduit dans la colonne (120), de préférence sensiblement à mi-hauteur. Il subit dans cette colonne une distillation d'équeutage, conduisant à une récupération en tête de colonne (120), d'hydroquinone purifiée *HQ* (122), avantageusement munie d'un reflux (123). Les impuretés lourdes sont quant à elles, éliminées en pied de colonne (120) sous la forme d'un flux (124) symbolisé par (II) sur la figure 2.

Ainsi, le procédé illustré sur la Figure 2 permet la séparation efficace, sous forme isolée et purifiée (donc valorisable), des deux constituants principaux (pyrocatéchol et hydroquinone) contenus dans les milieux réactionnels issus d'une réaction d'hydroxylation de phénol par du peroxyde d'hydrogène en présence d'un catalyseur acide. Ce procédé peut en outre être conduit selon un mode continu. Ce mode de réalisation particulier du procédé constitue un objet spécifique de la présente invention.

L'invention sera encore davantage illustrée par l'exemple ci-après qui décrit, de façon non limitative, un mode de réalisation possible de l'invention.

### EXEMPLE

On a utilisé un dispositif tel que représenté sur la Figure 1, employé en continu, pour effectuer la purification d'une hydroquinone brute contenant, en masse par rapport à la masse totale de l'hydroquinone brute, 0,6 % de résorcinol et 0,7 % de pyrogallol.

Ce mélange a été introduit dans la première colonne de distillation (10) avec un débit d'alimentation constant de 100 kg/h.

La colonne (10) utilisée présente les caractéristiques suivantes :
- nombre d'étages théoriques : 30
- température en tête de colonne : 202°C .
- pression de travail : 87 hPa
- taux de reflux : 600

Le temps de séjour de l'hydroquinone dans la colonne (10) est évalué à 25 min.

En tête de la colonne (10), on a obtenu un flux (I) comprenant du résorcinol, le résorcinol ayant un débit de 1 kg/h.

La deuxième colonne (20), dans laquelle le mélange brut obtenu en pied de la première colonne (10) a été introduit, a quant à elle, été utilisée dans les conditions suivantes :
- nombre d'étages théoriques : 30
- température en tête de colonne : 201 °C
- pression de travail : 73 hPa
- taux de reflux : 7

Le temps de séjour de l'hydroquinone dans la colonne (20) est évalué à 30 min.

En pied de la colonne (20), le flux (24) comprenant le pyrogallol a un débit de 1 kg/h.

En tête de la colonne (20), on a obtenu une hydroquinone purifiée *HQ* sortant avec un débit de 98 kg/h.

Cette hydroquinone comprend moins de 2000 ppm de résorcinol et moins de 200 ppm de pyrogallol.

Elle a donc une pureté de 99,78 % en masse.

L'hydroquinone obtenue présente une coloration de 30 Hazen.

## Revendications

1. Procédé de purification d'une hydroquinone brute *HQ⁰* contenant essentiellement de l'hydroquinone, et de faibles quantités d'impuretés incluant à la fois :
(i) des impuretés ayant une température de vaporisation inférieure à celle de l'hydroquinone, dites *impuretés légères,* incluant du résorcinol ; et
(ii) des impuretés ayant une température de vaporisation supérieure à celle de l'hydroquinone, dites *impuretés lourdes,* incluant du pyrogallol,
ledit procédé étant **caractérisé par le fait qu'**il comprend les étapes suivantes :
(A) une distillation d'étêtage, dans laquelle on injecte l'hydroquinone brute *HQ⁰* dans une colonne de distillation opérant avec un taux de reflux compris entre 300 et 2000 et où on élimine le résorcinol en tête de distillation éventuellement conjointement à tout ou partie des autres impuretés légères, ce par quoi on récupère en pied de colonne un mélange brut M comprenant de l'hydroquinone et les impuretés lourdes ; et
(B) une distillation d'équeutage dans laquelle on injecte le mélange brut M obtenu dans l'étape (A) dans une colonne de distillation et où on élimine le pyrogallol en pied de colonne éventuellement conjointement à tout ou partie des autres impuretés lourdes, ce par quoi on récupère en tête de colonne de l'hydroquinone sous une forme purifiée *(HQ)* comprenant moins de 4000 ppm d'impuretés.

2. Procédé selon la revendication 1, **caractérisé par le fait que** l'hydroquinone brute *HQ⁰* contient de 96 à 99,5 % en masse d'hydroquinone, et de 0,5 et 4 % en masse d'impuretés.

3. Procédé selon la revendication 1 ou selon la revendication 2, **caractérisé par le fait que** l'hydroquinone brute *HQ⁰* contient de 0,1 à 2 %, par exemple de 0,2 à 1 % en masse d'impuretés légères.

4. Procédé selon l'une des revendications 1 à 3, **caractérisé par le fait que** l'hydroquinone brute *HQ⁰* contient de 0,1 à 2 %, par exemple de 0,2 à 1 % en masse d'impuretés lourdes.

5. Procédé selon l'une des revendications 1 à 4, **caractérisé par le fait que** les impuretés légères présentes dans l'hydroquinone brute *HQ⁰* comprennent au moins 50 %, de préférence au moins 70 % de résorcinol, par rapport à la masse totale des impuretés légères.

6. Procédé selon l'une des revendications 1 à 5, **caractérisé par le fait que** les impuretés lourdes présentes dans l'hydroquinone brute *HQ⁰* comprennent au moins 50 %, de préférence au moins 70 % de pyrogallol, par rapport à la masse totale des impuretés lourdes.

7. Procédé selon l'une des revendications 1 à 6, **caractérisé par le fait que** l'hydroquinone brute *HQ⁰* est susceptible d'être obtenue à partir d'un mélange réactionnel issu d'une hydroxylation de phénol par le peroxyde d'hydrogène en présence de catalyseurs acides, après élimination substantielle du pyrocatéchol par distillation.

8. Procédé selon l'une des revendications 1 à 7, **caractérisé par le fait que** l'hydroquinone brute *HQ⁰* comprend, en masse par rapport à la quantité totale d'hydroquinone brute :
- de 96 à 99,5 % d'hydroquinone,
- de 0,1 à 2 %, de préférence de 0,2 à 1 % de résorcinol,
- de 0,1 à 2 %, de préférence de 0,2 à 1 % de pyrogallol
- éventuellement du pyrocatéchol sous forme de traces.

9. Procédé selon l'une des revendications 1 à 8, **caractérisé par le fait que**, dans l'étape (A), le point d'alimentation où est introduit l'hydroquinone brute *HQ⁰* est sensiblement à mi-hauteur de la colonne de distillation, avec un rapport volumique de la zone de rectification de la colonne de l'étape (A) à la zone d'épuisement de la colonne de l'étape (A) compris entre 25:75 et 75 : 25, plus préférentiellement entre 30 : 70 et 70 : 30.

10. Procédé selon l'une des revendications 1 à 9, **caractérisé par le fait que** le nombre d'étages théoriques de la colonne utilisée dans l'étape (A) est d'au moins 20, de préférence entre 30 et 50.

11. Procédé selon l'une des revendications 1 à 10, **caractérisé par le fait que** le temps de séjour de l'hydroquinone dans la colonne de l'étape (A) est compris entre 10 minutes et 1 heure, de préférence entre 15 et 30 minutes.

12. Procédé selon l'une des revendications 1 à 11, **caractérisé par le fait que**, dans l'étape (B), le point d'alimentation où est introduit le mélange brut M est sensiblement à mi-hauteur de la colonne de distillation, avec un rapport volumique de la zone de rectification de la colonne de l'étape (B) à la zone d'épuisement de la colonne de l'étape (B) compris entre 25 : 75 et 75 : 25, plus préférentiellement entre 30 : 70 et 70 : 30.

13. Procédé selon l'une des revendications 1 à 12, **caractérisé par le fait que** le flux qui sort en tête de la colonne de distillation de l'étape (B), est dérivé en partie, pour être réinjecté dans la colonne de distillation, avec un taux de reflux compris entre 1 et 15.

14. Procédé selon l'une des revendications 1 à 13, **caractérisé par le fait que** le nombre d'étages théoriques de la colonne utilisée dans l'étape (B) est d'au moins 20, de préférence entre 30 et 50.

15. Procédé selon l'une des revendications 1 à 14, **caractérisé par le fait que** le temps de séjour de l'hydroquinone dans la colonne de l'étape (B) est compris entre 10 minutes et 1 heure, de préférence entre 15 et 30 minutes.

16. Procédé selon l'une des revendications 1 à 15, **caractérisé par le fait que** les étapes (A) et (B) sont conduites sous une atmosphère inerte sensiblement exempte d'oxygène, de préférence sous argon ou sous azote.

17. Procédé selon l'une des revendications 1 à 16, **caractérisé par le fait que** les étapes (A) et (B) sont conduites à des pressions comprises entre 50 et 100, de préférence entre 60 et 90 hPa.

18. Procédé selon l'une des revendications 1 à 17, **caractérisé par le fait que** l'hydroquinone purifiée *HQ* obtenue à l'état liquide à l'issue des étapes (A) et (B) est refroidie, et mise en forme à l'état d'objets solides ayant une taille de l'ordre d'au moins quelques centaines de microns à quelques millimètres.

## Claims

1. Process for the purification of a crude hydroquinone *HQ°* essentially comprising hydroquinone and small amounts of impurities, including both:
(i) impurities having a lower evaporation temperature than that of hydroquinone, referred to as *light impurities,* including resorcinol; and
(ii) impurities having a higher evaporation temperature than that of hydroquinone, referred to as *heavy impurities,* including pyrogallol,
the said process being **characterized in that** it comprises the following stages:
(A) a topping distillation, in which the crude hydroquinone *HQ°* is injected into a distillation column operating with a reflux ratio of between 300 and 2000 and where the resorcinol is removed as distillation top product, optionally in conjunction with all or part of the other light impurities, whereby a crude mixture M, comprising hydroquinone and the heavy impurities, is recovered at the column bottom; and
(B) a tailing distillation, in which the crude mixture M obtained in stage (A) is injected into a distillation column and where the pyrogallol is removed at the column bottom, optionally in conjunction with all or part of the other heavy impurities, whereby hydroquinone in a purified form (*HQ*) comprising less than 4000 ppm of impurities is recovered at the column top.

2. Process according to Claim 1, **characterized in that** the crude hydroquinone *HQ⁰* comprises from 96 to 99.5% by weight of hydroquinone and from 0.5 to 4% by weight of impurities.

3. Process according to Claim 1 or according to Claim 2, **characterized in that** the crude hydroquinone *HQ⁰* comprises from 0.1 to 2% by weight, for example from 0.2 to 1% by weight, of light impurities.

4. Process according to one of Claims 1 to 3, **characterized in that** the crude hydroquinone *HQ⁰* comprises from 0.1 to 2% by weight, for example from 0.2 to 1% by weight, of heavy impurities.

5. Process according to one of Claims 1 to 4, **characterized in that** the light impurities present in the crude hydroquinone *HQ⁰* comprise at least 50% of resorcinol, preferably 70% of resorcinol, with respect to the total weight of the light impurities.

6. Process according to one of Claims 1 to 5, **characterized in that** the heavy impurities present in the crude hydroquinone *HQ⁰* comprise at least 50% of pyrogallol, preferably at least 70% of pyrogallol, with respect to the total weight of the heavy impurities.

7. Process according to one of Claims 1 to 6, **characterized in that** the crude hydroquinone *HQ⁰* is capable of being obtained from a reaction mixture resulting from a hydroxylation of phenol by hydrogen peroxide in the presence of acid catalysts, after substantial removal of the pyrocatechol by distillation.

8. Process according to one of Claims 1 to 7, **characterized in that** the crude hydroquinone *HQ⁰* comprises, by weight with respect to the total amount of crude hydroquinone:
- from 96 to 99.5% of hydroquinone,
- from 0.1 to 2%, preferably from 0.2 to 1%, of resorcinol,
- from 0.1 to 2%, preferably from 0.2 to 1%, of pyrogallol,
- optionally pyrocatechol in the form of traces.

9. Process according to one of Claims 1 to 8, **characterized in that**, in stage (A), the feed point where the crude hydroquinone *HQ⁰* is introduced is substantially at mid-height in the distillation column, with a ratio by volume of the rectification region of the column of stage (A) to the stripping region of the column of stage (A) of between 25:75 and 75:25, more preferably between 30:70 and 70:30.

10. Process according to one of Claims 1 to 9, **characterized in that** the number of theoretical stages of the column used in stage (A) is at least 20, for example between 30 and 50.

11. Process according to one of Claims 1 to 10, **characterized in that** the residence time of the hydroquinone in the column of stage (A) is between 10 minutes and 1 hour, preferably between 15 and 30 minutes.

12. Process according to one of Claims 1 to 11, **characterized in that**, in stage (B), the feed point where the crude mixture M is introduced is substantially at mid-height in the distillation column, with a ratio by volume of the rectification region of the column of stage (B) to the stripping region of the column of stage (B) of between 25:75 and 75:25, more preferably between 30:70 and 70:30.

13. Process according to one of Claims 1 to 12, **characterized in that** the stream which exists at the top of the distillation column of stage (B) is partially diverted, in order to be reinjected into the distillation column, with a reflux ratio of between 1 and 15.

14. Process according to one of Claims 1 to 13, **characterized in that** the number of theoretical stages of the column used in stage (B) is at least 20, for example between 30 and 50.

15. Process according to one of Claims 1 to 14, **characterized in that** the residence time of the hydroquinone in the column of stage (B) is between 10 minutes and 1 hour, preferably between 15 and 30 minutes.

16. Process according to one of Claims 1 to 15, **characterized in that** stages (A) and (B) are carried out under an inert atmosphere substantially devoid of oxygen, for example under argon or under nitrogen.

17. Process according to one of Claims 1 to 16, **characterized in that** stages (A) and (B) are carried out at pressures of between 50 and 100 hPa, preferably between 60 and 90 hPa.

18. Process according to one of Claims 1 to 17, **characterized in that** the purified hydroquinone *HQ* obtained in liquid state on conclusion of stages (A) and (B) is cooled and formed in the form of solid objects having a size of the order of at least a few hundred microns to a few millimeters.

## Patentansprüche

1. Verfahren zur Reinigung eines rohen Hydrochinons *HQ⁰,* das im Wesentlichen Hydrochinon und kleine Mengen an Verunreinigungen enthält, die gleichzeitig Folgendes beinhalten:
(i) Verunreinigungen mit einer niedrigeren Verdampfungswärme als das Hydrochinon, die als *niedrigsiedende Verunreinigungen* bezeichnet werden, darunter Resorcin, und
(ii) Verunreinigungen mit einer höheren Verdampfungswärme als das Hydrochinon, die als *hochsiedende Verunreinigungen* bezeichnet werden, darunter Pyrogallol,
wobei das Verfahren durch die folgenden Schritte gekennzeichnet ist:
(A) eine Topping-Destillation, wobei man das rohe Hydrochinon *HQ⁰* in eine Destillationskolonne injiziert, die bei einer Rückflussrate zwischen 300 und 2000 arbeitet, und wobei man das Resorcin am Kopf der Destillation, gegebenenfalls zusammen mit den gesamten oder einem Teil der anderen niedrigsiedenden Verunreinigungen, beseitigt, wodurch man im Sumpf der Kolonne ein rohes Gemisch M gewinnt, das Hydrochinon und die hochsiedenden Verunreinigungen umfasst, und
(B) eine Tailing-Destillation, bei der man das im Schritt (A) erhaltene rohe Gemisch M in eine Destillationskolonne injiziert und wobei man pyrogallol im Sumpf der Kolonne, gegebenenfalls zusammen mit den gesamten oder einem Teil der anderen hochsiedenden Verunreinigungen, beseitigt, wodurch man im Kopf der Kolonne Hydrochinon in gereinigter Form (*HQ*) gewinnt, das weniger als 4000 ppm an Verunreinigungen umfasst.

2. Verfahren nach Anspruch 1, **dadurch gekennzeichnet, dass** das rohe Hydrochinon *HQ⁰* von 96 bis 99,5 Massen-% Hydrochinon und von 0,5 bis 4 Massen-% an Verunreinigungen enthält.

3. Verfahren nach Anspruch 1 oder nach Anspruch 2, **dadurch gekennzeichnet, dass** das rohe Hydrochinon *HQ⁰* von 0,1 bis 2 Massen-%, zum Beispiel von 0,2 bis 1 Massen-%, an niedrigsiedenden Verunreinigungen enthält.

4. Verfahren nach einem der Ansprüche 1 bis 3, **dadurch gekennzeichnet, dass** das rohe Hydrochinon *HQ⁰* von 0,1 bis 2 Massen-%, zum Beispiel von 0,2 bis 1 Massen-%, an hochsiedenden Verunreinigungen enthält.

5. Verfahren nach einem der Ansprüche 1 bis 4, **dadurch gekennzeichnet, dass** die in dem rohen Hydrochinon *HQ⁰* vorliegenden niedrigsiedenden Verunreinigungen mindestens 50%, vorzugsweise mindestens 70% an Resorcin, bezogen auf die Gesamtmasse der niedrigsiedenden Verunreinigungen, umfassen.

6. Verfahren nach einem der Ansprüche 1 bis 5, **dadurch gekennzeichnet, dass** die in dem rohen Hydrochinon *HQ⁰* vorliegenden hochsiedenden Verunreinigungen mindestens 50%, vorzugsweise mindestens 70% an Pyrogallol, bezogen auf die Gesamtmasse der hochsiedenden Verunreinigungen, umfassen.

7. Verfahren nach einem der Ansprüche 1 bis 6, **dadurch gekennzeichnet, dass** das rohe Hydrochinon *HQ⁰* aus einem Reaktionsgemisch erhalten werden kann, das aus einer Hydroxylierung von Phenol mit Wasserstoffperoxid in Gegenwart saurer Katalysatoren stammt, nachdem Brenzcatechin durch Destillation im Wesentlichen beseitigt wurde.

8. Verfahren nach einem der Ansprüche 1 bis 7, **dadurch gekennzeichnet, dass** das rohe Hydrochinon *HQ⁰* in Massen-%, bezogen auf die Gesamtmenge an rohem Hydrochinon, Folgendes umfasst:
- von 96 bis 99,5 % Hydrochinon,
- von 0,1 bis 2 %, vorzugsweise von 0,2 bis 1 % Resorcin,
- von 0,1 bis 2 %, vorzugsweise von 0,2 bis 1 % Pyrogallol,
- gegebenenfalls Spuren von Brenzcatechin.

9. Verfahren nach einem der Ansprüche 1 bis 8, **dadurch gekennzeichnet, dass** im Schritt (A) der Beschickungspunkt, an dem das rohe Hydrochinon *HQ⁰* eingebracht wird, sich im Wesentlichen in der Mitte der Höhe der Destillationskolonne befindet, wobei das Volumenverhältnis der Rektifikationszone der Kolonne von Schritt (A) zur Ablasszone der Kolonne von Schritt (A) zwischen 25:75 und 75:25, stärker bevorzugt zwischen 30:70 und 70:30 beträgt.

10. Verfahren nach einem der Ansprüche 1 bis 9, **dadurch gekennzeichnet, dass** die Anzahl der theoretischen Böden der Kolonne, die im Schritt (A) verwendet wird, mindestens 20, vorzugsweise zwischen 30 und 50 beträgt.

11. Verfahren nach einem der Ansprüche 1 bis 10, **dadurch gekennzeichnet, dass** die Verweildauer des Hydrochinons in der Kolonne von Schritt (A) zwischen 10 Minuten und 1 Stunde, vorzugsweise zwischen 15 und 30 Minuten beträgt.

12. Verfahren nach einem der Ansprüche 1 bis 11, **dadurch gekennzeichnet, dass** im Schritt (B) der Beschickungspunkt, an dem das rohe Gemisch M eingebracht wird, sich im Wesentlichen in der Mitte der Höhe der Destillationskolonne befindet, wobei das Volumenverhältnis der Rektifikationszone der Kolonne von Schritt (B) zur Ablasszone der Kolonne von Schritt (B) zwischen 25:75 und 75:25, stärker bevorzugt zwischen 30:70 und 70:30 beträgt.

13. Verfahren nach einem der Ansprüche 1 bis 12, **dadurch gekennzeichnet, dass** der Strom, der den Kopfraum der Destillationskolonne von Schritt (B) verlässt, zum Teil abgeleitet wird, um wieder in die Destillationskolonne injiziert zu werden, wobei die Rückflussrate zwischen 1 und 15 beträgt.

14. Verfahren nach einem der Ansprüche 1 bis 13, **dadurch gekennzeichnet, dass** die Anzahl der theoretischen Böden der Kolonne, die im Schritt (B) verwendet wird, mindestens 20, vorzugsweise zwischen 30 und 50 beträgt.

15. Verfahren nach einem der Ansprüche 1 bis 14, **dadurch gekennzeichnet, dass** die Verweildauer des Hydrochinons in der Kolonne von Schritt (B) zwischen 10 Minuten und 1 Stunde, vorzugsweise zwischen 15 und 30 Minuten beträgt.

16. Verfahren nach einem der Ansprüche 1 bis 15, **dadurch gekennzeichnet, dass** die Schritte (A) und (B) unter einer inerten, im Wesentlichen Sauerstoff-freien Atmosphäre, vorzugsweise unter Argon oder unter Stickstoff, durchgeführt werden.

17. Verfahren nach einem der Ansprüche 1 bis 16, **dadurch gekennzeichnet, dass** die Schritte (A) und (B) bei Drücken zwischen 50 und 100 hPa, vorzugsweise zwischen 60 und 90 hPa durchgeführt werden.

18. Verfahren nach einem der Ansprüche 1 bis 17, **dadurch gekennzeichnet, dass** das gereinigte Hydrochinon *HQ,* das im flüssigen Zustand nach den Schritten (A) und (B) erhalten wird, gekühlt und in die Form fester Objekte mit einer Größe in der Größenordnung von einigen hundert Mikrometern bis zu mehreren Millimetern gebracht wird.
